# EUROPEAN PATENT SPECIFICATION

(11) **EP 3 715 472 B1**
(45) Date of publication and mention of the grant of the patent: **26.07.2023**
(21) Application number: 19164820.3
(22) Date of filing: 25.03.2019
(51) Int. Cl.: C12Q 1/6883

(54) **PHOSPHATE AND TENSIN HOMOLOG (PTEN) FOR THE DETECTION OF AUTOIMMUNE DISEASES OR CONDITIONS**
PHOSPHAT- UND TENSIN-HOMOLOG (PTEN) ZUM NACHWEIS VON AUTOIMMUNKRANKHEITEN
HOMOLOGUE DE PHOSPHATE ET TENSINE (PTEN) POUR LA DÉTECTION DE MALADIES AUTO-IMMUNES

(43) Date of publication of application: 30.09.2020
(73) Proprietor: Universitätsmedizin der Johannes Gutenberg-Universität Mainz, 55131 Mainz (DE)
(72) Inventor: ABEL, Janine, 65189 Wiesbaden (DE)
(74) Representative: Patentanwälte Dr. Keller, Schwertfeger Partnerschaft mbB

(56) References cited:
- WO-A1-2017/025533
- WO-A2-2006/130560
- STEVEN Z. JOSEFOWICZ ET AL: "Regulatory T Cells: Mechanisms of Differentiation and Function", ANNUAL REVIEW OF IMMUNOLOGY, vol. 30, no. 1, 23 April 2012 (2012-04-23) , pages 531-564, XP055240580, ISSN: 0732-0582, DOI: 10.1146/annurev.immunol.25.022106.141623

## Description

### FIELD OF THE INVENTION

The present invention relates to phosphate and tensin homolog (PTEN) and its use for the detection of autoimmune diseases.

### DESCRIPTION OF THE BACKGROUND ART

Naturally occurring CD4+ CD25+ regulatory T-cells (Treg) represent a unique T-cell lineage that is endowed with the ability to actively suppress immune responses. Treg represent less than 2 % of all peripheral T-cells in healthy individuals (Jonuleit H. et al. Identification and functional characterisation of human CD4+ CD25+ T-cells with regulatory properties isolated from peripheral blood. J Exp Med. 2001; 193:1285-1294). As immune modulators, Treg play a critical role in the maintenance of a peripheral immunologic tolerance by suppressing the activation of other immune cells and conferring regulatory properties upon suppressed T effector cells (Jonuleit et al., Infectious tolerance human CD25(+) regulatory T-cells convey suppressor activity to conventional CD4(+) T helper cells. J.Exp Med. 2002, 196.255-260; Stassen M. et al. Human CD25+ regulatory T-cells: two subsets defined by the integrins alpha 4 beta 7 or alpha 4 beta 1 confer distinct suppressive properties upon CD4+T helper cells. Eur J Immunol. 2004; 34:1303-1311). An impaired responsiveness of CD4+ T-cells to regulatory T-cells (Treg) is also known as Treg resistance. Treg resistance describes a condition of T cells in an inflammatory environment in which their sensitivity to suppressive mechanisms mediated by regulatory T cells is clearly disturbed. Treg resistance is detectible in a number of inflammatory and non-inflammatory autoimmune diseases. While the suppressive function of Treg depends on the activation, once activated, Treg suppress other immune cells in a non-specific manner resulting in a broad and systemic immunoregulatory effect (Thornton et al., CD4+CD25+ immunoregulatory T-cells suppress polyclonal T cell activation in vitro by inhibiting interleukin 2 production. J Exp Med. 1998, 188:287-296). The activation of autoreactive T-cells is controlled by regulatory T-cells (Treg) under physiological conditions (Jonuleit et al., 2001. Identification and functional characterization of human CD4(+)CD25(+)T-cells with regulatory properties isolated from peripheral blood. J Exp.Med. 193:1285-1924; Josefowicz et al, 2012. Regulatory T-cells: mechanisms of differentiation and function. Annual review of immunology 30:531-564; Sakaguchi et al., 2009. Regulatory T-cells: how do they suppress immune responses?

International immunology 21:1105-1111). Due to their unique function to regulate innate and adaptive immune responses, Treg have become a major subject in immunological research. As such, Treg play a major role in a number of autoimmune conditions or diseases such as rheumatoid arthritis, rheumatic fever, systemic lupus erythematosus (SLE), ulcerative colitis, Crohn's disease, autoimmune inflammatory bowel disease, diabetes type I, multiple sclerosis (MS), myasthenia gravis, psoriasis, pemphigus vulgaris, pemphigoid (Kelchtermans et al., Defective CD4+ CD25+ regulatory T cell functioning in collagen-induced arthritis: an important factor in pathogenesis, counter-regulated by endogenous IFN-gamma. Arthr res ther., 2005, 7:r402-r415; 14. Mcgeachy et al., Natural recovery and protection from autoimmune encephalomyelitis: contribution of CD4+ CD25+ regulatory cells within the central nervous system. J Immunol. 205, 175:3025-3032 15; Powrie et al., Phenotypically distinct subsets of CD4+ t cells induce or protect from chronic intestinal inflammation in C. B-17 scid mice, 1993, Int. Immunol. 5:1461-1471; Belkaid et al., CD4+ CD25+ regulatory T cells control Leishmania major persistence and immunity. Nature 2002, 420:502-507).

Among these diseases, multiple sclerosis (MS) is difficult to treat because as a heterogeneous autoimmune disease only a small portion of patients respond to therapy. New therapeutic treatments are difficult to investigate, although the therapeutically success has been significantly improved in the recent years. It is known so far that Treg are functionally impaired in MS patients (Baecher-Allan and Hafler, 2004b. Suppressor T-cells in human diseases, J Exp.Med. 200:273-276; Costantino et al., 2008. Multiple sclerosis and regulatory T-cells. J Clin Immunol 28:697-706; Haas et al., 2005. Reduced suppressive effect of CD4+CD25high regulatory T-cells on the T-cell immune response against myelin oligodendrocyte glycoprotein in patients with multiple sclerosis. European journal of immunology 35:3343-3352; Schneider et al., 2013, "In active relapsing-remitting multiple sclerosis, effector T-cell resistance to adaptive T(regs) involves IL-6-mediated signalling", Sci.Transl.Med. 5:170ra115; Trinschek et al., 2013 Kinetics of IL-6 production defines T effector cell responsiveness to regulatory T-cells in multiple sclerosis. PloS one 8:e77634; Zozuyla and Siendl, 2008. The role of regulatory T-cells in multiple sclerosis. Nat. Clin.Pract.Neurol. 4:384-398). Due to this imbalance, autoreactive T-cells escape suppression by Treg and migrate through the blood brain barrier to attack the central nervous system (Goverman, 2009. Autoimmune T-cell responses in the central nervous system. Nature reviews. Immunology 9:393-407).The cytokine IL-6 plays a major role by inducing an enhanced PKB/c-Akt phosphorylation, upregulated IL-6R expression and in a positive feedback loop its own accelerated production (Trinschek et al., 2013. Kinetics of IL-6 production defines T-effector cell responsiveness to regulatory T-cells in multiple sclerosis. PloS one 8:e77634).

It is desirable to distinguish between defective Treg and Treg-resistant T-cells in patients with autoimmunity. This differentiation is complicated by the fact that both T-cell populations do not differ in their expression of certain activation markers or their cytokine profiles (Ferrante et al., 1998. Cytokine production and surface marker expression in acute and stable multiple sclerosis: altered IL-12 production and augmented signaling lymphocytic activation molecule (SLAM)-expressing lymphocytes in acute multiple sclerosis. Journal of Immunology (Baltimore, Md. : 1950) 160:1514-1521; Vladic et al., 2002. Cerebrospinal fluid and serum protein levels of tumour necrosis factor-alpha (TNF-alpha) interleukin-6 (IL-6) and soluble interleukin-6 receptor (sIL-6R gp80) in multiple sclerosis patients. Cytokine 20:86-89). These barriers increased the demand for the identification of molecules that are associated with impaired T-cell function and that provide important insights in the understanding of T-cell-Treg interaction (Havla et al., 2015. [Immunotherapies for multiple sclerosis : review and update]. Der Internist 56:432-445; Katsavos and Anagnostouli, 2013. Biomarkers in Multiple Sclerosis: An Up-to-Date Overview. Multiple sclerosis international 2013:340508; Weiner, 2009. The challenge of multiple sclerosis: how do we cure a chronic heterogeneous disease? Annals of neurology 65:239-248).

Treg resistance is strongly associated with an enhanced activation of proteinkinase B (PKB). It appears that the activation state of PKB pathway plays an important role in determining the sensitivity of T cells to Treg-mediated suppression. However, underlying mechanisms which lead to a stronger phosphorylation of PKB and thus Treg resistance in T effector cells are still unclear.

Biomarkers and methods that allow for the detection of impaired responsiveness of T-effector cells to Treg are described in WO 2017/025533 A1. They relate to the measurement of the levels of peroxisome proliferator-activated receptor gamma coactivator 1-alpha (PPARGC1A; PGC1-alpha).

WO 2006/130560A2 describes a method for modulating the expression of PTEN in Treg thereby modulating the immune response. The Treg response/activation is inversely associated with the expression of the phosphatase and PTEN.

Steven Z. Josefowicz et. al describe the pathway involves in the regulation of the activity of regulatory T-cells during inflammation or auto-immune disease ("Regulatory T Cells: Mechanisms of Differentiation and Function", Annual review of immunology, vol. 30, no. 1, 23 April 12 (2012-04-23), pages 531-564, XP055240580, ISSN: 0732-0582, DOl: 10.1146/annurev.immunol.25.022106.141623).

### SUMMARY OF THE INVENTION

Against this background it is the object of the present invention to provide an alternative biomarker that allows for the efficient detection of impaired responsiveness of T-effector cells to regulatory T-cells (Treg), and methods for the detection autoimmune diseases associated with Treg resistance.

This object is solved by the use of phosphatase and tensin homolog (PTEN) as novel biomarker and its use in methods for the detection of impaired responsiveness of T-effector cells to regulatory T-cells (Treg). Preferred embodiments are part of the dependent claims.

The methods of the invention relate to the detection of impaired responsiveness of CD4+ T-cells to regulatory T-cells (Treg), referred to as Treg resistance, by measuring the expression levels of phosphatase and tensin homolog (PTEN) in activated CD4+ T-cells. As shown herein, PTEN allows the detection of Treg resistance in isolated human or animal cells or cell cultures. It is known that Treg resistance is caused by increased IL-6 receptor expression, accelerated IL-6 production and an enhanced phosphorylation of PKB/c-Akt. Functional genetic expression profiles of Treg-resistant CD4+ T-cells from patients suffering of an autoimmune disease were generated to identify PTEN as biomarker associated with Treg resistance. As shown by the inventors, PTEN is associated with Treg resistance and provides an efficient diagnostic tool to monitor therapeutic success of a given treatment. PTEN is closely linked to the IL-6 pathway, which plays a major role by inducing an enhanced PKB/c-Akt phosphorylation and up-regulated IL-6 R expression. It has been demonstrated herein that a downregulation of PTEN is associated with the induction of Treg resistance. The inventors modified PTEN expression in Teff from healthy donors using PTEN-specific siRNA. PTEN knockdown in Teff resulted in an accelerated phosphorylation of PKB and an impaired responsiveness towards Treg-mediated suppression, comparable to Treg-resistant Teff from MS patients. As a consequence, autoimmune diseases can be personalized by addressing specific molecules leading to reduced side effects, thereby enhancing therapy efficiency and success.

In a first aspect of the invention, PTEN is suitable as a biomarker for the detection of Treg resistance and hence the detection of an autoimmune disease associated with Treg resistance. Patients with multiple sclerosis (MS) were compared with healthy individuals in a study that compares the expression profiles of PTEN in activated T-cells. Accordingly, effector T-cells from MS-patients show a strongly reduced expression level compared to T-cells from healthy individuals.

Since T-cell susceptibility to Treg correlates with PTEN expression levels, the expression of PTEN is down-regulated in response to IL-6 as well as in activated T-cells of autoimmune patients and expression levels correlated with Treg-resistance. Since PTEN is down-regulated in autoimmune patients, a therapy could be directed to the up-regulation of PTEN in activated T-cells in order to improve susceptibility to Treg function.

The inventors further found that PTEN down-regulation is directly inducible by IL-6, thereby providing promising approaches for the development of new therapeutic strategies. As part of the therapeutic approach, agonists that can up-regulate PTEN expression in activated T-cells of autoimmune patients can be identified.

The detection of Treg resistance is preferably carried out by comparing the expression levels of PTEN with the respective biomarker expression levels of activated Treg-sensitive CD4+ T-cells, wherein a down-regulation of PTEN within activated Treg-resistant T-cells as compared to the activated Treg-sensitive CD4+ and CD8+ T-cells is indicative for Treg resistance. In the methods according to the present invention, up-regulation of PTEN is correlated with a responsiveness of Treg-sensitive CD4+ T-cells to Treg-mediated suppression. As demonstrated herein, a down-regulation of PTEN is associated with an accelerated IL-6 production following T cell receptor (TCR) stimulation, enhanced phosphorylation of PKB/c-Akt and/or an increased IL-6 receptor (IL-6R) expression. PTEN is also one of the most commonly lost tumor suppressors in human cancer, in fact, during tumor development, mutations and deletions of PTEN occur in up to 70% of men with prostate cancer leading to increased proliferation of cancer cells due to hyperactivation of the PKB signaling pathway. In the case of autoimmune diseases such as MS, a pronounced hyperactivation of PKB can be observed.

The present invention also concerns an *in-vitro* screening method for the detection of an inflammatory disease comprising the steps of generating a functional gene expression profile by measuring the expression levels of phosphatase and tension homolog (PTEN) in Treg-resistant CD4+ T-cells from patients suffering of an autoimmune disease and comparing the obtained gene expression profile with the expression profile from Treg-sensitive CD4+T-cells from healthy controls. In particular, the screening method of the invention allows an early detection of a number of autoimmune diseases such as multiple sclerosis when comparing the expression profiles in activated T-cells of MS patients to the expression profiles of healthy individuals. In a further aspect of the invention, the screening method of the invention can be used for the detection of similar Treg-resistance-associated autoimmune diseases such as rheumatoid arthritis, rheumatic fever, systemic lupus erythematosus (SLE), ulcerative colitis, Crohn's disease, autoimmune inflammatory bowel disease, diabetes type I, myasthenia gravis, psoriasis, pemphigus vulgaris, pemphigoid. As further demonstrated, amelioration of T-cell function correlates with a normalization of PTEN expression, indicating that a restauration of Treg activity is a promising therapeutic approach for treatment of autoimmune disorders such as multiple sclerosis or any other of the mentioned autoimmune disorders. The transition from Treg-resistant affected T-cells to Treg-sensitive affected T-cells correlates with the expression levels of PTEN. In addition, IL-6 expression levels increase significantly in activated T-cells from MS patients in which PTEN is down-regulated. On the other side, IL-6R expression is significantly reduced and PTEN is up-regulated in activated T-cells of healthy individuals. As mentioned before, down-regulation of PTEN in activated MS T-cells is linked to an enhanced PKB/c-Akt phosphorylation and unresponsiveness of T-cells to Treg mediated suppression.

The following examples will demonstrate the invention in more detail.

### Examples:

First, the inventors investigated the expression of PTEN, which negatively regulates PKB activation. MS was used as a model disease but the results are also applicable to other autoimmune disorders.

Treg-resistant CD4+CD25- Teff from MS patients were stimulated with anti-CD3/CD28 mAb and PTEN mRNA expression was analysed by qRT-PCR. Treg-sensitive Teff from healthy donors served as controls. PTEN mRNA was significantly downregulated after activation in Teff from MS patients compared to healthy controls. To further clarify if downregulation of PTEN is associated with the induction of Treg resistance, the inventors modified PTEN expression in Teff from healthy donors using PTEN-specific siRNA. PTEN knockdown in Teff resulted in an accelerated phosphorylation of PKB and an impaired responsiveness towards Treg-mediated suppression, comparable to Treg-resistant Teff from MS patients.

The inventors report for the first time about a disturbed PTEN expression in Teff from MS patients after TCR-mediated stimulation, indicating that PTEN and PKB are two important key players for the induction of Treg resistance in MS.

### Treg resistance is directly linked to IL-6 and PKB signaling pathway

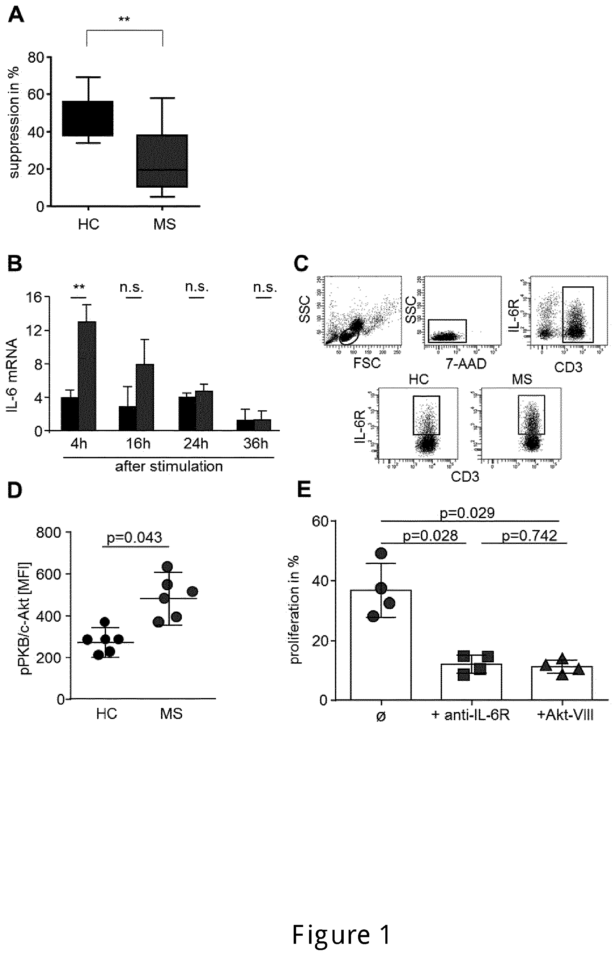

Treg resistance refers to a condition of T cells in an inflammatory environment in which their sensitivity to suppressive mechanisms mediated by regulatory T cells is clearly disturbed (Figure 1A). It has previously been shown that Treg-resistant CD4+ T cells from therapy-naive MS patients are characterized by an accelerated IL-6 mRNA production (Figure 1B), increased IL-6 receptor expression (Figure 1C) and stronger phosphorylation of PKB/c-Akt (Figure 1D). Direct inhibition of either PKB activity (via Akt VIII) or IL-6 signaling pathway (via anti-IL-6 receptor antibody) restores responsiveness towards Treg-mediated suppression (Figure 1E).

### PKB as a central mediator to define responsiveness towards regulatory T cells

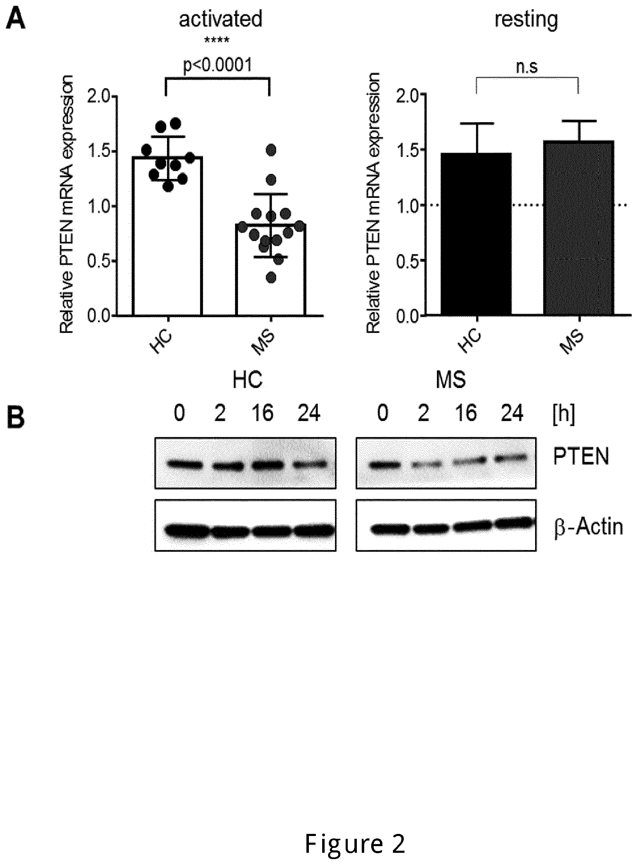

Since the activation state of PKB/c-Akt seems to be a critical factor for T cell responsiveness towards Treg-mediated suppression, PTEN which regulates activation and inactivation of PKB/c-Akt, was further investigated as a potential candidate. PTEN dephosphorylates PIP3 to PIP2 which results in inhibition of the AKT signaling pathway. To determine possible reasons for this hyperactivation, a disturbed regulation of PKB in Treg resistant T cells from MS patients was hypothesized. PTEN expression in T cells was investigated from healthy controls and therapy-naive MS patients in the resting state as well as shortly after stimulation with anti-CD3 and anti-CD28 mAb. PTEN mRNA expression was significantly reduced in T cells from MS patients compared to healthy controls 2 hours after polyclonal stimulation. No difference in total PTEN mRNA expression was observed in resting T cells (Figure 2A). Furthermore, a significantly different kinetic of PTEN protein expression was observed in T cells of MS patients compared to healthy controls (Figure 2B). In MS, PTEN protein expression is strongly downregulated already 2 hours after polyclonal activation whereas in T cells from healthy donors, no significant regulation of PTEN protein expression could be observed.

### Disturbed PTEN expression in MS T cells is associated with Treg resistance

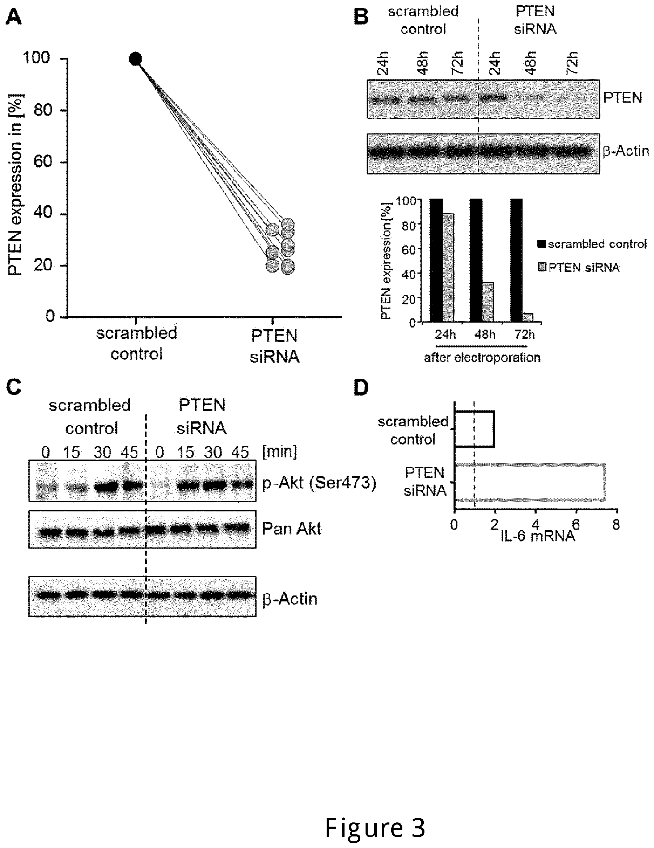

To clarify the role of PTEN protein in T cell proliferation and suppression PTEN knockdown experiments were performed in CD4⁺ T cells of healthy donors. Freshly isolated untouched CD4⁺CD25⁻ T cells were successfully electroporated with PTEN-specific siRNA. 24 hours after electroporation, PTEN knockdown efficiency was analyzed on mRNA and protein level. PTEN mRNA as well as protein expression was significantly reduced (up to 80%) after treatment with specific siRNA (Figures 3A+B). Polyclonal stimulation of knockdown T cells resulted in an accelerated phosphorylation of PKB/c-Akt as well as IL-6 mRNA production compared to scrambled control (3C+D), both important characteristics which are associated with Treg resistance in MS T cells.

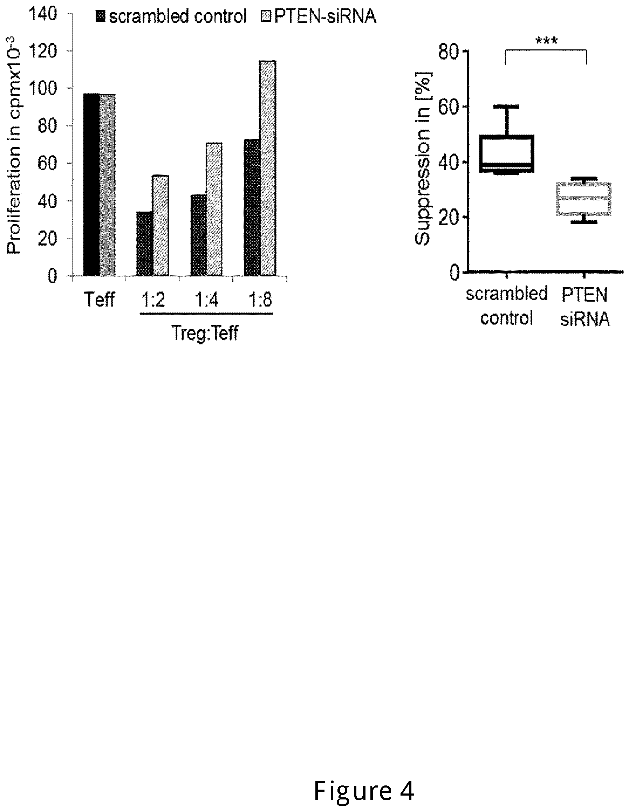

Next, in vitro T cell suppression was investigated. Therefore, T cells were co-cultured alone or in the presence of different Treg ratios following polyclonal stimulation with anti-CD3 mAb. T cell-depleted PBMC were used as APC to stimulate CD28 receptor. T cell proliferation was measured via incorporation of 3H-Tdr. In vitro results showed a clearly reduced susceptibility of PTEN knockdown T cells towards Treg-mediated suppression compared to scrambled controls (Figure 4). Targeted knockdown of PTEN leads to Treg resistance in T cells from healthy donors, which, as in MS, is associated with accelerated activation of PKB and IL-6 mRNA production. Thus, it can be assumed that the disturbed PTEN expression in T cells of MS patients strongly correlates with their impaired responsiveness towards Treg-mediated suppression.

### IL-6, as a second important key player in Treg resistance, influences PTEN protein expression in T cells

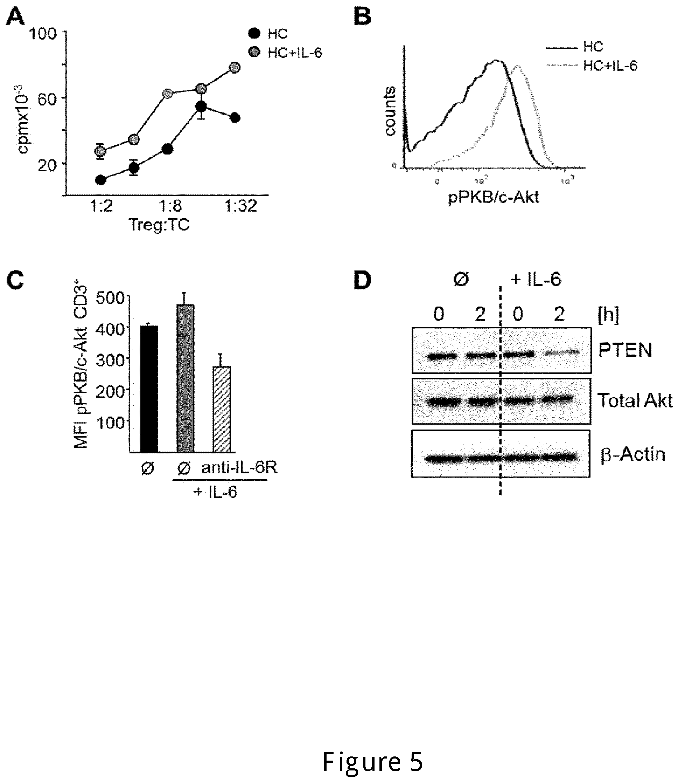

In addition to PKB, also IL-6 and its signaling pathway play a crucial role in whether T cells from MS patients respond to suppression by Treg or not. In the context of autoimmune diseases, it has been investigated how the increased IL-6 production observed in MS can affect PTEN protein expression. Therefore, CD4+CD25- T cells from healthy donors were first incubated overnight with increased concentrations of IL-6 to induce Treg resistance (Figure 5A). This induced Treg resistance correlated, similar to MS, with an increased phosphorylation of protein kinase B (Figure 5B). Hyperactivation of PKB was directly linked to IL-6 since blocking of the IL-6 signaling pathway with an anti-IL-6 receptor mAb abolished this effect (Figure 5C). Polyclonal activation of IL-6-treated T cells resulted in a significant decrease in PTEN protein expression 2 hours after activation compared to untreated controls (Figure 5D). IFN-β improves responsiveness of T cells in RRMS patients for immunoregulation by Treg.

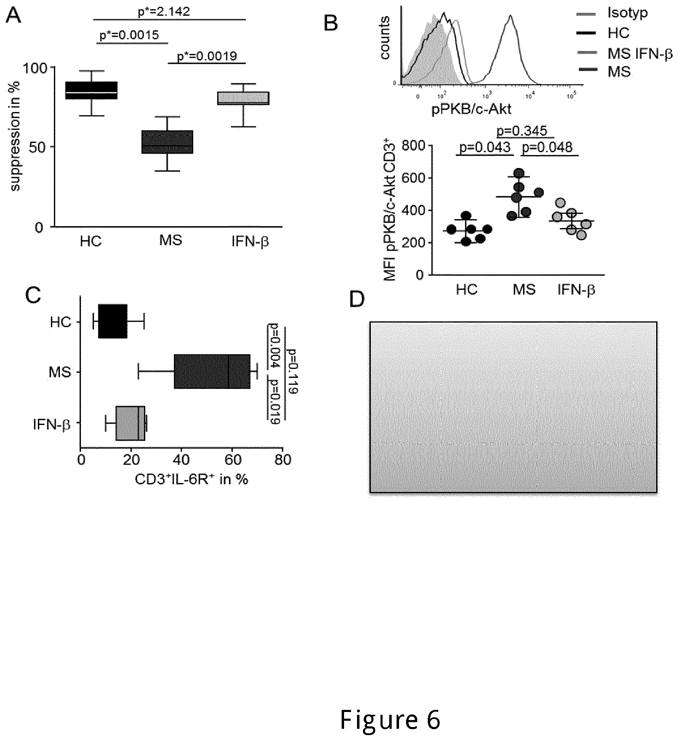

The inventors were able to show that IFN-β therapy of MS patients restores susceptibility of CD4+ T cells towards Treg-mediated immunoregulation (Figure 6A). This improved responsiveness of T cells in turn correlated with a normalization of PKB activity and IL-6 receptor expression similar to healthy controls (Figures 6B + 6C). In a next step, it was analyzed to which extent IFN-β therapy might also affect PTEN protein expression and regulation. Therefore, untouched CD4+CD25- T cells were again isolated and PTEN mRNA and protein expression was investigated upon TCR-mediated activation (Figure 6D).

### Figure Legends

**Figure 1****: Treg resistance is directly linked to IL-6 and PKB signaling pathway**
   A) PBMC from therapy-naïve MS patients (red) or HC (black) were cocultured with Treg and stimulated with anti-CD3 mAb. T cell proliferation was determined by ³H-Tdr incorporation on day three. Box plots show percentage of suppression in presence of Treg (ratio 4:1) normalized to proliferation of T cells alone as median with interquartile range (n=15), P-values relative to T cells of HC are shown, to avoid familywise error rate bonferroni correction was used p*. B) C) IL-6R expression within CD3+ T cells of PBMC from HC or MS was determined by flow cytometry. One representative of eight independent experiments is shown. D) PKB/c-Akt phosphorylation was determined by flow cytometry within CD3⁺ T cells of MS (red) or HC (black). Six different experiments are shown, p-values relative to MFI of HC. E) MS T cells with Treg were stimulated with anti-CD3 mAb without (circle) or with (quadrats) anti-IL-6R mAb or (triangles) Akt-VIII inhibitor. Each dot represents percentage of proliferation with Treg normalized to T cells alone (n=4), p-values to MS T cells.
**Figure 2****: Dysregulated PTEN expression in Treg resistant MS T cells**
   A) Left: Relative PTEN mRNA expression in CD4⁺ T cells from healthy donors (black) and therapy-naive MS patients (red) 2 hours after activation with 0.5 µg/ml anti-CD3 and 1 µg/ml anti-CD28 mAb. Every dot represents one individual donor (healthy n=9, MS n=13). Right: Relative PTEN mRNA expression in resting CD4⁺ T cells from healthy donors (black) and therapy-naive MS patients (red). Bars show mean PTEN mRNA expression of n= 12 individuals. B) Western blot depicts total PTEN protein expression of CD4⁺ T cells from healthy donors (left) and therapy-naïve MS patients (right) at different time points after activation with 0.5 µg/ml anti-CD3 and 1 µg/ml anti-CD28 mAb.
**Figure 3****: PTEN knockdown results in an accelerated IL-6 mRNA production and activation of PKB**
   A) Relative PTEN mRNA expression in CD4⁺ T cells 24h after electroporation with 1 µM PTEN siRNA normalized to scrambled control. Every dot represents one individual knockdown experiment (n=9). Knockdown efficiency ranges from 70-80%. B) Western blot depicts total PTEN protein expression of electroporated T cells at different time points after electroporation. C) After electroporation with PTEN siRNA and scrambled control, CD4⁺ T cells were activated and PKB/c-Akt phosphorylation was analyzed. Western Blot shows phosphorylation state of PKB/c-Akt (Ser473) at different time points (0-45 min) after activation. D) 24h after electroporation CD4⁺ T cells were activated with 0.5 µg/ml anti-CD3 and 1 µg/ml anti-CD28 mAb for 2 hours. Bars show relative IL-6 mRNA expression in CD4⁺ T cells treated with scrambled control (black) and PTEN siRNA (red) 2h after activation normalized to unstimulated controls.
**Figure 4****: PTEN knockdown induces Treg resistance in CD4⁺ T cells**
   A) 24h after electroporation CD4⁺ T cells were cocultured with freshly isolated Treg and activated with 0.1 µg/ml anti-CD3 mAb. CD3-depleted PBMC served as costimulus. Proliferation of T cells was measured via ³H-Tdr incorporation. Left graph shows suppression of electroporated T cells of one representative experiment (n=5). Box plots show percentage of suppression in presence of Treg (T cell:Treg ratio 4:1) of all 5 experiments, black: scrambled control, grey: PTEN siRNA.
**Figure 5****: IL-6 mediates Treg resistance by inducing the phosphorylation of PKB/c-Akt and downregulation of PTEN in activated T cells** A) T cells and Treg were cocultured in presence (grey) or absence (black) of IL-6 and stimulated with anti-CD3 mAb. Proliferation was determined by ³H-Tdr incorporation on day three and displayed as mean ± SEM of triplicate measurements, n=4. B) PKB/c-Akt phosphorylation was analyzed by flow cytometry within T cells after 24 h of culture with (black) or without IL-6 (grey). C) T cells from HC were incubated for 24 h without IL-6 (black) or with IL-6 (grey) or IL-6 and anti-IL-6R mAb (striped). Shown is the mean fluorescence intensity (MFI) of pPKB/c-Akt in T cells (n=5). D) PBMC from HC were cultured for 24 h in the presence or absence of 500 ng/ml IL-6. CD4⁺ T cells were isolated, left unstimulated or were activated with anti-CD3 and anti-CD28 mAb for two hours. Total proteins were isolated and PTEN protein expression was analyzed via western blot.
**Figure 6****: Treg resistance of T cells from MS patients is ameliorated after IFN-β therapy**
   A) Treg-depleted PBMC from therapy-naive (red), IFN-β-treated MS patients (blue) or HC (black) were cocultured with allogeneic Treg and stimulated with anti-CD3 mAb. T cell proliferation was determined by ³H-Tdr incorporation on day three. Box plots show percentage of suppression in presence of Treg (ratio 1:1) normalized to proliferation of PBMC alone as median with interquartile range (n=15), p-values relative to suppression of HC or therapy-naive MS, to avoid familywise error rate bonferroni correction was used indicated as (p*). B) PKB/c-Akt phosphorylation was determined by flow cytometry within CD3⁺ T cells from therapy-naive (red), IFN-β-treated MS patients (blue) or HC (black). Grey histogram depicts isotypic control of MS. Lower panel shows MFI of PKB/c-Akt phosphorylation of six different experiments, p-values relative to MFI of MS. C) IL-6R expression within PBMC from HC (black), therapy-naive (red), or IFN-β-treated (blue) MS patients was determined by flow cytometry. Box plots show percentage of IL-6R⁺ cells within CD3⁺ T cells of six independent donors, p-values relative to IL-6R expression of therapy-naive MS or HC are shown. D) Analysis of PTEN expression in CD4⁺ T cells from healthy donors, therapy-naive MS patients and IFN-β-treated (or DMF) MS patients.

### Materials and Methods

### Isolation and culture of human immune cells

PBMC from either MS patients or healthy donors were isolated from buffy coats or heparinized syringes within 12 h after blood collection as described before using density gradient centrifugation. Blood was kept at room temperature before PBMC enrichment. After isolation, human cells were cultured in X-VIVO-15 (Lonza, Belgium).

### Flow cytometry

Flow cytometric analysis was performed using the following antibodies: anti-human CD3 (UCHT1), anti-human CD4 (RPA-T4), anti-human CDS (SK1), anti-human CD14 (M5E2), anti-human CD19 (HIB19), all from BD Pharmingen, anti-human CD25 (4E3), anti-human CD127 (MB15-18C9), all from Miltenyi Biotec. Fluorokine^{®} biotinylated human Interleukin-6 (R&D systems) was used to analyze IL-6R expression on T cells. Cell viability during flow cytometric analysis was determined using 7-AAD and eFluor506 (eBioscience). For blockade experiments, cells were incubated with a neutralizing antibody against anti-IL-6R (Tocilizumab). For surface staining of PBMC or T cells indicated antibodies were incubated for 20 min at 4 °C and washed twice with PBS supplemented with 0.5% HSA + 1 mM EDTA + 10 µg/ml Sandoglobulin (CSL Behring). Stained cells were measured on LSRII with FACS Diva Software (BD Bioscience) or FACSVia^{™} and data was analyzed with BD FACSVia Research Software (BD Biosciences). To detect phosphorylated PKB/c-Akt, cells were fixed at 37 °C with BD Cytofix Buffer for 10 min, then permeabilized with BD Phosflow Perm Buffer III for 30 min on ice, washed twice with BD Stain Buffer and stained with anti-Akt pS473 (BD Phosflow) according to manufacturer's instructions.

### Isolation of T cell subsets

Untouched CD4⁺CD25⁻ T cells were isolated using CD4⁺ T cell isolation kit (Miltenyi Biotec) according to manufacturer's instructions. For some experiments, CD4⁺ T cells were enriched by positive selection using anti-CD4 MicroBeads (Miltenyi Biotec). Isolated T cells were then depleted of intrinsic Treg with anti-CD25 Dynabeads (Invitrogen). CD4⁺CD25⁺Foxp3⁺ Treg were isolated from PBMC using anti-CD25 MicroBeads (Miltenyi Biotec) and depleted of contaminating CD8⁺, CD14⁺ and CD19⁺ cells with Dynabeads (Invitrogen) as described previously. Purity was routinely >85 %, Treg functionality was ensured in standard suppressor assays. For some experiments PBMC were depleted of CD3 or CD25 using corresponding Dynabeads (1 bead/cell; Invitrogen).

### Suppressor assays

CD4⁺CD25⁻ T cells (10⁵ cells/well, 96 well plate, flat bottom) were stimulated with 0.1 µg/ml anti-CD3 mAb (OKT-3) and cultured in presence or absence of different Treg ratios. CD3-depleted PBMC (5×10⁴/well) were used as costimulus. For some experiments, CD25-depleted PBMC (10⁵ cells) were stimulated with 0.1 µg/ml anti-CD3 mAb (OKT-3) and co-cultured with or without different Treg ratios. On day 3, ³H-Tdr was added to each well (37 kBq/well) and cells were cultured for an additional 16 h. T cell proliferation was measured by ³H-Tdr incorporation using a liquid β-scintillation counter. Some experiments were performed by supplementing cultures with neutralizing mAb against IL-6R (30 ng/ml; Tocilizumab; Roacterma; Roche) or supplemented with IL-6 (500 ng/ml, ImmunoTools)

### RNA isolation, cDNA synthesis and qRT-PCR

RNA was extracted from 1-2×10⁶ cells using peqGOLD Micro RNA Kit (VWR) according to manufacturer's instructions. cDNA was synthesized from 100 ng of the isolated RNA by reverse transcription with iScript^{™} cDNA synthesis kit (Bio-Rad) and the supplied random hexamer primers. All quantitative RT-PCR reactions were performed in triplicates on a Rotor-Gene Q cycler (Qiagen) using SYBR Green (Bimake). PTEN and IL-6 mRNA expression levels were measured with the appropriate QuantiTect Primer Assay (Qiagen) according to manufacturer's protocol. The mRNA levels of the housekeeping gene EEF1A were used for normalization and relative expression levels were calculated with 2^{-ΔΔCT}method.

### PTEN knockdown experiments

For a successful knockdown of PTEN protein, PTEN GeneSolution siRNA kit (Qiagen) was used that includes Hs_PTEN_6, Hs_PTEN_9, Hs_PTEN_8, and Hs_PTEN_4 siRNAs.

AllStars Negative Control siRNA (Qiagen) was employed as scrambled control. Purified CD4⁺ T cells were transfected with AMAXA^{®} Human T cell Nucleofector^{®} kit (Lonza) according to manufacturer's protocol. Briefly, 5×10⁸ T cells were resuspended in 100 µl Nucleofector^{®} Solution (per sample) supplemented with 1 µM PTEN siRNA mix or negative control siRNA. Nucleofector program U-014 was used to efficiently transfect T cells. After nucleofection, T cells were cultured overnight in X-VIVO-15 in a humidified 37°C/5% CO₂ incubator and knockdown efficiency was analyzed at indicated time points using qRT-PCR and Western blot.

### SDS-PAGE and Western Blotting

Cell pellets were lysed in Cell Extraction Buffer (Invitrogen) supplemented with 1 mM PMSF (Invitrogen) and Protease Inhibitor Cocktail (Sigma) for 30 min on ice with vortexing at 10 min intervals. Total protein concentration of cell lysates was determined with Micro BCA Protein Assay Kit (Thermo Fisher Scientific) according to manufacturer's protocol.

Protein separation was performed using the NuPAGE^{®} Bis-Tris electrophoresis system (Thermo Fisher Scientific). Briefly, up to 15 µg of total protein were mixed with NuPAGE LDS Sample Buffer and NuPAGE Reducing Agent and loaded onto NuPAGE 4-12% (gradient) Bis-Tris gels (denaturing conditions, 200 V, 60 min, with NuPAGE MOPS SDS Running Buffer). The separated proteins were transferred onto PVDF membranes (0.45 µm pore size, Novex) using the semi-wet XCell II^{™} Blotting system (30 V, 60 min, Invitrogen). For immunoblot analysis, membranes were blocked in TBST buffer/5% BSA for at least 1 h at room temperature. The incubation with primary antibodies was performed as suggested by the antibody providers. For primary incubation, the following antibodies were used: rabbit anti-PTEN mAB (138G6), rabbit anti-β-Actin mAb (13E5), rabbit anti-pan-Akt mAb (C67E7), all from Cell Signaling Technology, mouse anti-PKB/Akt pSer437 mAb (11E6, Nanotools). Purified goat anti-rabbit IgG antibody (Cell Signaling Technology) and rabbit anti-mouse IgG antibody (Abeam) conjugated to horseradish peroxidase were employed for chemiluminescent detection with Amersham ECL Prime Western Blotting Detection Reagent (GE Healthcare Life Sciences). Western blots were analyzed and quantified with ImageJ software.

## Claims

1. A method for the detection of impaired responsiveness of CD4+T-cells to regulatory T-cells (Treg), referred to as Treg resistance, by measuring the expression levels of phosphatase and tensin homolog (PTEN) in activated CD4+ T-cells ex-vivo, wherein the expression levels of PTEN are compared between activated CD4+ T-cells from patients suffering of an autoimmune disease and activated Treg-sensitive CD4+ and CD8+ T-cells from healthy donors, wherein a downregulation of PTEN within activated CD4+ T-cells as compared to the activated Treg-sensitive CD4+ T-cells is indicative for Treg resistance, and wherein an upregulation of PTEN is correlated with a responsiveness of activated CD4+ T-cells to Treg-mediated suppression.

2. The method according to claim 1, wherein the Treg resistance correlates with an accelerated IL-6 production after T cell receptor (TCR) stimulation, enhanced phosphorylation of PKB/c-Akt and/or an increased IL-6 receptor (IL-6R) expression.

3. The method according to any claim 1 or claim 2, wherein impaired responsiveness of CD4+T-cells is restored by normalizing PTEN expression in activated Treg-resistant T-cells with IFN-β or dimethyl fumarate (DMF).

4. An *in vitro* screening method for the detection of an autoimmune disease comprising the steps of generating a functional gene expression profile by measuring the expression levels of phosphatase and tensin homolog (PTEN) in activated CD4+ T-cells from patients suffering of an autoimmune disease and comparing the obtained gene expression profile with the expression profile from Treg-sensitive CD4+ T-cells from healthy controls.

5. The method according to claim 4, wherein the generation of a functional gene expression profile comprises the steps of:
a. extraction and purification of RNA from T-cells,
b. synthesis of cDNA from the RNA templates,
c. labelling of cDNA with a marker molecule and hybridization to a gene chip,
d. analysis of the gene chips by detecting the intensity of the marker molecule.

6. The method according to claim 4 or claim 5, wherein impaired responsiveness of CD4+ and/or CD8+ T-cells to regulatory T-cells (Treg) is detected by a method according to any one of claims 1 to 3.

7. The method according to any one of claims 4 to 6, wherein the autoimmune disease is rheumatoid arthritis, rheumatic fever, systemic lupus erythematosus (SLE), ulcerative colitis, Crohn's disease, autoimmune inflammatory bowel disease, diabetes type I, multiple sclerosis (MS), myasthenia gravis, psoriasis, pemphigus vulgaris, pemphigoid.

## Patentansprüche

1. Verfahren zum Nachweis einer beeinträchtigten Reaktionsfähigkeit von CD4+ T-Zellen auf regulatorische T-Zellen (Treg), die als Treg-Resistenz bezeichnet wird, durch Messung der Expressionsniveaus von Phosphatase und Tensin Homolog (PTEN) in aktivierten CD4+ T-Zellen ex-vivo, wobei die Expressionsniveaus von PTEN zwischen aktivierten CD4+ T-Zellen von Patienten, die an einer Autoimmunerkrankung leiden, und aktivierten Treg-sensitiven CD4+ und CD8+ T-Zellen von gesunden Spendern verglichen werden, wobei eine Herabregulierung von PTEN innerhalb aktivierter CD4+ T-Zellen im Vergleich zu den aktivierten Treg-sensitiven CD4+ T-Zellen auf eine Treg-Resistenz hinweist, und wobei eine Hochregulierung von PTEN mit einer Ansprechbarkeit aktivierter CD4+ T-Zellen auf Treg-vermittelte Suppression korreliert ist.

2. Verfahren nach Anspruch 1, wobei die Treg-Resistenz mit einer beschleunigten IL-6-Produktion nach T-Zell-Rezeptor (TCR)-Stimulation, einer erhöhten Phosphorylierung von PKB/c-Akt und/oder einer erhöhten IL-6-Rezeptor (IL-6R)-Expression korreliert.

3. Verfahren nach einem der Ansprüche 1 oder 2, wobei die beeinträchtigte Reaktionsfähigkeit von CD4+T-Zellen durch Normalisierung der PTEN-Expression in aktivierten Treg-resistenten T-Zellen mit IFN-β or Dimethylfumarat (DMF) wiederhergestellt wird.

4. In-vitro-Screening-Verfahren zum Nachweis einer Autoimmunerkrankung, umfassend die Schritte des Erstellens eines funktionellen Genexpressionsprofils durch Messen der Expressionsniveaus von Phosphatase und Tensin Homolog (PTEN) in aktivierten CD4+ T-Zellen von Patienten, die an einer Autoimmunerkrankung leiden, und Vergleichen des erhaltenen Genexpressionsprofils mit dem Expressionsprofil von Treg-sensitiven CD4+ T-Zellen von gesunden Kontrollpersonen.

5. Verfahren nach Anspruch 4, wobei die Erstellung eines funktionellen Genexpressionsprofils die folgenden Schritte umfasst:
a. Extraktion und Reinigung von RNA aus T-Zellen,
b. Synthese von cDNA aus den RNA-Vorlagen,
c. Markierung der cDNA mit einem Markermolekül und Hybridisierung auf einen Genchip,
d. Analyse der Genchips durch Nachweis der Intensität des Markermoleküls.

6. Verfahren nach Anspruch 4 oder Anspruch 5, wobei eine beeinträchtigte Reaktionsfähigkeit von CD4+ und/oder CD8+ T-Zellen auf regulatorische T-Zellen (Treg) durch ein Verfahren nach einem der Ansprüche 1 bis 3 nachgewiesen wird.

7. Verfahren nach einem der Ansprüche 4 bis 6, wobei die Autoimmunerkrankung rheumatoide Arthritis, rheumatisches Fieber, systemischer Lupus erythematodes (SLE), Colitis ulcerosa, Morbus Crohn, autoimmune entzündliche Darmerkrankung, Diabetes Typ I, Multiple Sklerose (MS), Myasthenia gravis, Psoriasis, Pemphigus vulgaris, Pemphigoid ist.

## Revendications

1. Procédé pour la détection de la réponse altérée de cellules CD4+ T à des cellules T régulatrices (Treg), appelée la résistance aux Treg, en mesurant les niveaux d'expression d'un homologue de phosphate et de tensine (PTEN) dans des cellules CD4+ T activées ex-vivo, dans lequel les niveaux d'expression du PTEN sont comparés entre des cellules CD4+ T activées issues de patients souffrant d'une maladie auto-immune et des cellules CD4+ et CD8+ T sensibles aux Treg activées issues de donneurs sains, dans lequel une régulation à la baisse du PTEN à l'intérieur des cellules CD4+ T activées par comparaison avec les cellules CD4+ T sensibles aux Treg activées est indicative d'une résistance aux Treg, et dans lequel une régulation à la hausse du PTEN est corrélée à une réponse des cellules CD4+ T activées à une suppression à médiation par Treg.

2. Procédé selon la revendication 1, dans lequel la résistance aux Treg présente une corrélation avec une production d'IL-6 accélérée après une stimulation de récepteurs de cellules T (TCR), une phosphorylation accrue de PKB/c-Akt et/ou une expression augmentée de récepteurs d'IL-6 (IL-6R).

3. Procédé selon l'une quelconque de la revendication 1 ou de la revendication 2, dans lequel la réponse altérée de cellules CD4+ T est restaurée en normalisant l'expression du PTEN dans des cellules T résistant aux Treg activées à l'aide d'IFN-β ou de fumarate de diméthyle (DMF).

4. Procédé de recherche par criblage in vitro pour la détection d'une maladie auto-immune, comprenant les étapes de génération d'un profil d'expression fonctionnelle des gènes en mesurant les niveaux d'expression d'un homologue de phosphate et de tensine (PTEN) dans des cellules CD4+ T activées issues de patients souffrant d'une maladie auto-immune et de comparaison du profil d'expression des gènes obtenu avec le profil d'expression découlant de cellules CD4+ T sensibles aux Treg provenant de contrôles sains.

5. Procédé selon la revendication 4, dans lequel la génération d'un profil d'expression fonctionnelle des gènes comprend les étapes comprenant :
a. l'extraction et la purification d'ARN issu de cellules T ;
b. la synthèse de cADN issu des matrices d'ARN ;
c. le marquage de cADN à l'aide d'une molécule marqueur et l'hybridation en lien avec une puce à ADN ; et
d. l'analyse des puces à ADN en détectant l'intensité de la molécule marqueur.

6. Procédé selon la revendication 4 ou la revendication 5, dans lequel la réponse altérée de cellules CD4+ et/ou CD8+ T à des cellules T régulatrices (Treg) est détectée au moyen d'un procédé selon l'une quelconque des revendications 1 à 3.

7. Procédé selon l'une quelconque des revendications 4 à 6, dans lequel la maladie auto-immune est l'arthrite rhumatoïde, le rhumatisme articulaire aigu, le lupus érythémateux disséminé (SLE), la rectocolite hémorragique, la maladie de Crohn, la maladie inflammatoire auto-immune de l'intestin, le diabète de type I, la sclérose en plaques (MS), la myasthénie grave, le psoriasis, le pemphigus vulgaire et la pemphigoïde.
